# EUROPÄISCHE PATENTSCHRIFT

(11) **EP 1 585 715 B1**
(45) Veröffentlichungstag und Bekanntmachung des Hinweises auf die Patenterteilung: **16.12.2009**
(21) Anmeldenummer: 04701599.5
(22) Anmeldetag: 13.01.2004
(51) Int. Cl.: C07C 51/43, C07C 51/42, B01J 14/00

(54) **AUFREINIGUNG EINES MONOMERS DURCH EXTRAKTION MIT EINEM PHASENBILDNER UND KRISTALLISATION**
PURIFICATION OF A MONOMER BY EXTRACTION WITH A PHASE-SEPARATING AGENT AND CRYSTALLISATION
PURIFICATION D'UN MONOMERE PAR EXTRACTION AU MOYEN D'UN GENERATEUR DE PHASES ET CRISTALLISATION

(30) Priorität: 13.01.2003 DE 10301040
(43) Veröffentlichungstag der Anmeldung: 19.10.2005
(73) Patentinhaber: Evonik Stockhausen GmbH, 47805 Krefeld (DE)
(72) Erfinder: BALDUF, Torsten, Houston, Tx 77062-2133 (US); NORDHOFF, Stefan, 45657 Recklinghausen (DE); THONG YU-CHIANG, Dennis, 45772 Marl (DE); KOBUS, Axel, 44797 Bochum (DE); ROOS, Martin, 45721 Haltern (DE)
(74) Vertreter: Lang, Arne
(86) Internationale Anmeldenummer: PCT/EP2004/000163
(87) Internationale Veröffentlichungsnummer: WO 2004/063134

(56) Entgegenhaltungen:
- EP-A- 1 002 787
- DE-C- 863 050
- US-A- 3 663 375
- US-B1- 6 174 929
- US-B1- 6 448 439

## Beschreibung

Die Erfindung betrifft ein Verfahren zur Aufreinigung eines eine Doppelbindung aufweisenden, sauren Monomers sowie eine Vorrichtung zur Synthese eines eine Doppelbindung aufweisenden, sauren Monomers.

Auf Grund der Tatsache, dass auf Monomere basierende Massenkunststoffe heutzutage zunehmend mehr in Bereichen eingesetzt werden, bei denen in puncto Hygiene und Sortenreinheit der Kunststoffe an diese hohe Anforderungen gestellt werden, kommt der Bereitstellung von reinen Monomeren zur großtechnischen Kunststoffsynthese eine immer größere Bedeutung zu.

Diesen zunehmend in den Vordergrund drängenden Anforderungen an die Reinheit der Monomere wird durch entsprechend verbesserte und zu höheren Reinheiten führenden Monomersynthesen in immer weiter verbesserten Reaktoren und mit neuen Katalysatorsystemen entsprochen.

Trotz der großen Anstrengungen auf dem Gebiet der Reaktor- und Katalysatorforschung sind die aus den Reaktoren kommenden Monomerrohströme immer noch so stark verunreinigt, dass diese Rohmonomerströme einer'sorgfältigen Reinigung unterzogen werden müssen, bevor das so erhaltene reine Monomer durch Polymerisation zu entsprechend reinen Kunststoffen weiter verarbeitet werden kann. Daher kommt der Aufreinigung der Rohmonomerströme bei der technischen Herstellung von Monomeren eine erhebliche Bedeutung zu.

Ein anderer zunehmend an Bedeutung gewinnender Parameter bei der Weiterentwicklung von technischen Synthesen von Monomeren und deren Aufarbeitung ist der Umweltaspekt. In diesem Zusammenhang kommt der Verwendung von Wasser bzw. von wässrigen Systemen eine große Bedeutung zu. Ferner trägt zur Umweltfreundlichkeit von Technischen Synthesen die Vermeidung von Abfällen sowie die Erzielung hoher Ausbeuten bei hohen Umsätzen bei. Daher ist es bei technischen Synthesen von Monomeren von Bedeutung auch in ihrem prozentuellen Anteil gering erscheinende Monomermengen, die sich in verschiedensten Abströmen der Technischen Monomersynthese bilden können, zurückzugewinnen und aufzuarbeiten, um auf diese Weise die Ausbeute der technischen Monomersynthese zu optimieren.

Ein Beispiel für den vorstehend beschriebenen Trend ist die Synthese von wasserabsorbierenden Polymeren, die in Hygieneartikeln wie Windeln, Damenbinden oder Inkontinenzartikeln eingesetzt werden. An diese wasserabsorbierenden Polymere werden sehr hohe Reinheitsanforderungen gestellt. Daher sind die Reinheitsanforderungen, die an die zur Herstellung dieser wasserabsorbierenden Polymere verwendete Acrylsäure gestellt werden, ebenfalls sehr hoch. Ein anderes Beispiel für technisch hergestellte Polymere an die hohe Reinheitsanforderungen gestellt werden ist der Bereich der zur Trinkwasseraufbereitung verwendeten Polymere. Dabei handelt es sich oftmals um nicht vernetzte, lineare Polyacrylsäuren, die auf Grund der hohen Reinheitsanforderungen aus möglichst reiner Acrylsäure hergestellt werden.

US 4,720,577 lehrt die Reinigung von niedermolekularen, keine Doppelbindung aufweisenden Karbonsäuren wie Essigsäure durch den Einsatz eines aus aliphatischen Aminen und Phenol gebildeten Extraktionshilfsmittel. Der Einsatz eines aus zwei Komponenten, dem Amin und dem Phenol bestehenden Extraktionshilfsmittels ist im Vergleich zu einem Einkomponenten-Extraktionshilfsmittel aufwendig.

US 3,997,599 offenbart die Aufreinigung von Karbonsäuren wie Methacrylsäure als wässrige Phase durch Extraktion mit Trioctylphosphinoxid in einem organischen Lösemittel wie Kerosin. Der Einsatz des zum einen die Umwelt belastenden und ferner sehr brandgefährlichen organischen Lösemittel ist nachteilhaft.

DE 21 36 396 lehrt eine Aufreinigung von Acrylsäure durch Gegenstromwäsche mit einem extrem hydrophoben Lösemittel, wobei als Lösemittel bevorzugt organische Lösemittel eingesetzt werden, die eine erhöhte Brandgefahr und Umweltbelastung mit sich bringen.

DE 863 050 beschreibt die Aufreinigung von wässriger Acryl- und Methacrylsäure durch Extraktion unter Zugabe von konzentrierter Schwefelsäure, wobei sich eine obere an Acryl- und Methacrylsäure reiche Phase ausbildet. In dieser Phase haben sich jedoch eine Reihe von Verunreinigungen wie Benzaldehyd, Propionsäure oder dimere Acryl- und Methacrylsäure ebenfalls gegenüber der eingesetzten Ausgangsmischung angereichert. Dieses ist für die großtechnische Anwendung dieser Extraktion nachteilhaft.

US 3,663,375 ist eine dreistufiges Verfahren zur Aufreinigung von einer Isobuttersäure und Methacrylsäure beinhaltenden Mischung durch Aussalzen mit Schwefelsäure oder Natriumsulfat offenbart, wobei in einer ersten Stufe diese Mischung unter Ausbildung einer wässrigen und einer organischen Phase ausgesalzt, in einem zweiten Stufe die Phasen von einander getrennt und in einer dritten Stufe die getrennten Phasen jeweils getrennt von einander durch Destillation aufgearbeitet werden. Diese Lehre ist zum einen wegen der vielen Stufen und zum anderen wegen der Monomere wie Acryl- und Methacrylsäure thermisch stark belastenden Destillation nachteilhaft. Bei der Destillation von Acryl- und Methacrylsäure kommt es besonders leicht zur Bildung von Acryl- und Methacrylsäuredimeren bzw. Oligomere, die bei der großtechnischen Weiterverarbeitung zu Polymeren unerwünscht sind.

DE 196 06 877 A1 offenbart die Extraktion eines bei der Acrylsäuresynthese anfallenden Sauerwassernebenstroms mit einem kleinen Teilstrom eines nahezu Acrylsäure-freien Lösemittels. Der Hauptstrom des durch Gasphasenoxidation erhaltenen Acrylsäuregasstroms wird zunächst einer Absorption mit einem schwersiedenden Lösemittel, gefolgt von einer Destillation und einer Kristallisation aufgearbeitet. Die hier offenbarte Extraktion ist durch den Einsatz des nahezu Acrylsäure-freien Lösemittels nachteilhaft und nicht für den Hauptstrom geeignet.

Der vorliegenden Erfindung liegt zum einen allgemein die Aufgabe zu Grunde, die sich aus dem Stand der Technik ergebenen Nachteile zu überwinden.

Zum anderen liegt eine erfindungsgemäße Aufgabe darin, die Gewinnung der synthetisierten Monomere aus einer Mischung, die neben den gewünschten synthetisierten Monomeren weitere Verunreinigungen enthält, möglichst kostengünstig und umweltschonend sowie mit großen Ausbeuten zu erhalten.

Weiterhin besteht eine erfindungsgemäße Aufgabe darin, die Aufreinigung von Monomeren mit einem aus möglichst wenigen Komponenten zusammengesetzten Phasenbildner zu ermöglichen.

Außerdem liegt eine erfindungsgemäße Aufgabe darin, die Aufreinigung eines Monomers über möglichst wenige Schritte zu erreichen.

Ferner liegt eine erfindungsgemäße Aufgabe darin, bei der Aufreinigung von Monomeren die dieser begleitenden verschiedenen Verunreinigungen möglichst gleichzeitig durch eine Reinigungsmaßnahme abzureichern.

Ferner liegt eine erfindungsgemäße Aufgabe darin, eine möglichst schonende Monomeraufreinigung bereitzustellen, da es sich bei den Monomeren meist um reaktive und thermisch wenig belastbare Verbindungen handelt.

Die vorstehenden Aufgaben werden durch die nachstehenden kategoriebildenden Ansprüche gelöst, wobei Unteransprüche zu diesen kategoriebildenden Ansprüchen bevorzugte erfindungsgemäße Ausführungsformen darstellen.

Insbesondere betrifft die Erfindung ein Verfahren zur Aufreinigung eines eine Doppelbindung aufweisenden, sauren Monomers, aufweisend die Schritte
(a) Bereitstellen einer Ausgangsmischung, beinhaltend als Ausgangsmischungsbestandteile, jeweils bezogen auf die Ausgangsmischung,
   (a1) mindestens 5 Gew.-%, vorzugsweise mindestens 30 Gew.-% und besonders bevorzugt mindestens 60 Gew.-% sowie darüber hinaus bevorzugt 62 bis 90 Gew.-% des sauren Monomers, und entweder
   (a2) mindestens 0,01 Gew.-%, vorzugsweise mindestens 1 Gew.-% und besonders bevorzugt mindestens 10 Gew.-% Wasser, oder
   (a3) mindestens 0,01 Gew.-%, vorzugsweise mindestens 0,1 und besonders bevorzugt mindestens 5 Gew.-% einer Ausgangsmischungskomponente, oder
      (a2) und (a3),
   wobei die Summe der Gew.-%-Anteile der Ausgangsmischungsbestandteile jeweils 100 Gew.-% ergibt;
(b) Zugabe eines, vorzugsweise im Vergleich zu dem sauren Monomer hygroskopischeren, Phasenbildners oder eines Salz dieses Phasenbildners oder einer Mischung von beiden unter Erhalt eines Reinigungsgemisches, woraus sich
(c) mindestens eine erste Phase und eine durch eine Phasengrenze von der ersten Phase abgegrenzte mindestens eine weitere Phase als ein Phasensystem ausbildet;
(d) Absenken der Temperatur des Phasensystems; wobei
(e) in einer der Phasen des Phasensystems ein mindestens 50 Gew.-%, vorzugweise mindestens 70 Gew.-% und besonders bevorzugt mindestens 90 Gew.-% sowie darüber hinaus bevorzugt mindestens 95 Gew.-%, eines der Ausgangsmischungsbestandteile, vorzugsweise das saure Monomer, beinhaltender Produktkristall neben einem andern Ausgangsmischungsbestandteil als ein Kristallsystem entsteht;
(f) Abtrennen der Produktkristalle.

In einer bevorzugten Ausführungsform des erfindungsgemäßen Verfahrens in erfolgen die Schritte (c) und (d) an einem gleichen Ort, vorzugsweise in einem gemeinsamen Behälter, einstufig. In einer anderen bevorzugten Ausführungsform des erfindungsgemäßen Verfahrens in erfolgen die Schritte (c) und (d) räumlich von einander getrennt, vorzugsweise in mindestens zwei Behälter, zwei- oder mehrstufig.

Weiterhin ist es in einer erfindungsgemäßen Ausführungsform bevorzugt, dass die Schritte (c) und (d) nicht durch weitere Schritte unterbrochen werden und daher unmittelbar aufeinander folgen.

In einer ferner bevorzugten erfindungsgemäßen Ausführungsform des Verfahrens weist die Ausgangsmischung als Ausgangsmischungsbestandteil (a3) mindestens 1 Gew.-%, vorzugsweise mindestens 15 Gew.-% und besonders bevorzugt mindestens 30 Gew.-% wenigstens einer Ausgangsmischungskomponente auf Dieses gilt insbesondere für verschieden Sümpfe einer Aufarbeitung des sauren Monmers. Bei der Ausgangsmischungskomponente, vorzugsweise der Ausgangsmischungskomponente (a3), handelt es sich vorzugsweise um von den sauren Monomeren verschiedene Verunreinigungen außer Wasser. Vorzugsweise besitzen die Ausgangsmischungskomponenten ein höheres Molekulargewicht als die erfindungsgemäßen Monomere. Weiterhin kann es bevorzugt sein, dass die Ausgangsmischungskomponenten um mindestens 5°C, vorzugsweise mindestens 20°C und besonders bevorzugt mindestens 40°C höher als das saure Monomer sieden. Oligomere des sauren Monomers bilden häufig eine typische Ausgangsmischungskomponente. Unter Oligomeren des sauren Monomers werden hier Moleküle verstanden, die aus mindestens zwei sauren Monomermolekülen bestehen. Weitere häufig in der Ausgangsmischung vorkommende Ausgangsmischungskomponenten sind die bei der Synthese und Aufarbeitung des sauren Monomers anfallenden Reaktionsprodukte, vorzugsweise wie unter den Beispielen nachfolgend beschrieben.

Zum einen fallen Ausgangsmischungen oftmals in Sümpfen von Destillationskolonnen bei der Herstellung von sauren Monomeren an. Eine solche Sumpf-Ausgangsmischung beinhaltet als Sumpf-Ausgangsmischungsbestandteile, jeweils bezogen auf die Sumpf-Ausgangsmischung,
(alS) mindestens 5 Gew.-%, bevorzugt mindestens 50 Gew.-% und besonders bevorzugt mindestens 75 Gew.-% eines sauren Monomers,
(a2S) mindestens 0,05 Gew.-%, bevorzugt mindestens 2 Gew.-% und besonders bevorzugt mindestens 5 Gew.-% Wasser,
(a3S) mindestens 1 Gew.-%, bevorzugt mindestens 5 Gew.-% und besonders bevorzugt mindestens 10 Gew.-% einer Ausgangsmischungskomponente,
wobei die Summe der Gew.-%-Anteile der Sumpf-Ausgangsmischungsbestandteile jeweils 100 Gew.-% ergibt.

Weiterhin fallen nach dem erfindungsgemäßen Verfahren gut zu reinigende Ausgangsgemische häufig an den Köpfen von Destillationskolonnen, in denen die sauren Monomere destilliert werden, an.

Eine solche Kopf-Ausgangsmischung beinhaltet als KopfAusgangsmischungsbestandteile, jeweils bezogen auf die Kopf-Ausgangsmischung,
(a1K)mindestens 0,5 Gew.-%, bevorzugt mindestens 50 Gew.-% und besonders bevorzugt mindestens 75 Gew.-% eines sauren Monomers,
(a2K)mindestens 0,05 Gew.-%, bevorzugt mindestens 2 Gew.-% und besonders bevorzugt mindestens 5 Gew.-% Wasser,
(a3K)mindestens 0,5 Gew.-%, bevorzugt mindestens 1 Gew.-% und besonders bevorzugt mindestens 2 Gew.-% einer Ausgangsmischungskomponente,
wobei die Summe der Gew.-%-Anteile der Kopf-Ausgangsmischungsbestandteile jeweils 100 Gew.-% ergibt.

Ferner fallen durch das erfindungsgemäße Verfahren vorteilhaft aufreinigbare Ausgangsmischungen bei der Oligomerenspaltung an. Diese Oligomere des sauren Monomers bilden sich an verschiedenen Stellen der Aufreinigung von sauren Monomere beinhaltenden Strömen. Die Oligomerbildung kann zudem bereits während der Synthese des sauren Monomers erfolgen. Ferner kann die Oligomerenbildung bei der thermischen Reinigung der sauren Monomere durch Destillation auftreten. Darüber hinaus ist oftmals zu beobachten, dass sich Oligomere des sauren Monomers in Reinigungsschritten wie der Kristallisation anreichern.

Eine solche aus einem Oligomerenspalter als Sumpf entstehende Oligomer-Ausgangsmischung beinhaltet die Oligomer-Ausgangsmischungsbestandteile wie zuvor für den Sumpf beschrieben.

Zudem ist die Aufreinigung von Mischungen, die mindestens ein saures Monomer und Wasser beinhalten, durch Kristallisation durch Phasendiagramme eingeschränkt. Um eine weitere Anreicherung von saurem Monomer in diesen mindestens ein saures Monomer und Wasser beinhaltenden Mischungen zu erreichen, kann das erfindungsgemäße Verfahren vorteilhaft eingesetzt werden.

Die Betrachtung des Phasendiagramms eines solchen mindestens binären, mindestens das saure Monomer als Hauptbestandteil und einen verglichen mit dem sauren Monomer nächst geringeren Nebenbestandteil, vorzugsweise Wasser, beinhaltenden Eutektikum-Ausgangsmischung weist Solidus- und Liquiduslinien in einem Temperatur/Konzentration-Phasendiagramm auf. Weitere Einzelheiten zur Bestimmung der Solidus- und Liquiduslinien in einem Temperatur/Konzentration-Phasendiagramm sind für verschiedenen Phasensysteme bei "Theory and Applicaton of Melt Crystallization" promoted by the European Thematic Network CRYSOPT, 28./29.09.2000 in Halle-Wittenberg Chapter 2 "*Phase Diagrams*" von Prof. Dr. Axel König, Universität Erlangen-Nürnberg mit weiteren Nachweisen beschrieben. Diese Solidus- und Liquiduslinien weisen mindestens einen S-L-Schnittpunkt auf, in dem sich die beiden Linien schneiden oder berühren. Charakteristische Phasendiagramme, durch die sich auf die erfindungsgemäßen beschreiben lassen, sind in Atsuoka, M.: "Developments in Melt Crystallization", Advances in "Industrial Crystallization", J. Garside; R.J. Davey and A.G. Jones, eds., Oxford (U.K.); Butterworth-Heinemann Ltd. (1991), pp. 229-244 beschrieben.

Eine erfindungsgemäß bevorzugte Eutektikum-Ausgangsmischung beinhaltet als Eutektikum-Ausgangsmischungsbestandteile, das saure Monomer in einem Konzentrationsbereich, der von dem mindestens einem S-L-Schnittpunkt +/- maximal 60 %, bevorzugt +/- maximal 30 %, vorzugsweise +/- maximal 10 % und besonders bevorzugt +/- maximal 5 %, sofern nicht Konzentrationen an saurem Monomer von weniger als 0 oder mehr als 100 % erreicht werden. Die entsprechenden Nebenkomponentenkonzentrationen ergeben sich entsprechend aus dem Phasendiagramm.

Bevorzugte Eutektikum-Ausgangsmisehungen-AA von Acrylsäure, Wasser und einer von Wasser verschiedenen einer Ausgangsmischungskomponente beinhalten als Eutektikum-Ausgangsmischungsbestandteile, jeweils bezogen auf die Eutektikum-Ausgangsmischung,
(a1E) im Bereich von 0,01 bis 99,99 Gew.-%, vorzugsweise in einem Bereich von 15 bis 85 Gew.-% und besonders bevorzugt in einem Bereich von 40 bis 65 Gew.-%, eines sauren Monomers,
(a2E) im Bereich von 5 bis 95 Gew.-%, vorzugsweise in einem Bereich von 10 bis 80 Gew.-% und besonders bevorzugt in einem Bereich von 30 bis 50 Gew.-%, Wasser,
(a3E) mindestens 0,01 Gew.-%, bevorzugt mindestens 2 Gew.-% und besonders bevorzugt mindestens 10 Gew.-% einer Ausgangsmischungskomponente,
wobei die Summe der Gew.-%-Anteile der Eutektikum-Ausgangsmischungsbestandteile jeweils 100 Gew.-% ergibt.

Weiterhin ist es in dem erfindungsgemäßen Verfahren bevorzugt, dass das Reinigungsgemisch den Phasenbildner in einer Menge im Bereich von 1 bis 80 Gew.-%, vorzugsweise im Bereich von 10 bis 50 Gew.-% und besonders bevorzugt im Bereich von 20 bis 30 Gew.-% aufweist.

Zudem ist es bevorzugt, das Salz des Phasenbildners als Lösungen einzusetzen, in denen Wasser das bevorzugte Lösemittel ist. Derartige Phasenbildnersalzlösungen beinhalten das Phasenbildnersalz in einer Konzentration im Bereich von 1 bis 60 Gew.-%, vorzugsweise im Bereich von 20 bis 55 Gew.-% und besonders bevorzugt im Bereich von 40 bis 50 Gew.-%, jeweils bezogen auf die Phasenbildnersalzlösung.

Außerdem ist es in dem erfindungsgemäßen Verfahren bevorzugt, dass eine Brönsted-Säure mit einem pH-Wert von weniger als 6, vorzugsweise weniger als 3 und besonders bevorzugt weniger 1, ein Salz dieser Brönsted-Säure oder eine Mischung daraus als Phasenbildner eingesetzt wird. Als Brönsted-Säuren sind insbesondere Säuren bevorzugt, die Atome der 5. und 6. Hauptgruppe des Periodensystems der Elemente, vorzugsweise Phosphor, Schwefel oder Sauerstoff, aufweisen. Dabei haben sich Schwefelsäure und Phosphorsäure sowie die Alkali- oder Erdalkalisalze der Schwefelsäure oder Phosphorsäure besonders bewährt, wobei Schwefelsäure, Natriumhydrogensulfat oder deren Mischungen bevorzugt sind.

Weiterhin ist es in dem erfindungsgemäßen Verfahren bevorzugt, dass der Phasenbildner bei der Zugabe flüssig ist. Daher sind Phasenbildner, die bei 20°C unter Normalbedingungen als Flüssigkeit vorliegen, besonders bevorzugt.

Unter "hygroskopisch" wird erfindungsgemäß das in Römpp, Lexikon der Chemie, 10. völlig überarbeitete Auflage 1997, Seite 1858 mit weiteren Nachweisen beschriebene und durch einen Hygrometer zu messende Affinität zu Wasser verstanden.

Der Umfang und die Art der Temperaturerniedrigung in dem erfindungsgemäßen Verfahren hängt von dem Verlauf der Liquidus- und Soliduslinien der jeweiligen Temperatur/Konzentration-Phasendiagramme der zu trennenden Ausgangsmischungen ab. So ist es bevorzugt, dass die Erniedrigung der Temperatur unterhalb der Liquiduslinie eines Ausgangsmischungsbestandteils in der Ausgangsmischung erfolgt. Ferner ist es bevorzugt, dass die Temperatur oberhalb der Soliduslinien gehalten wird. Zudem ist es bevorzugt, dass die Temperatur in dem von Solidus- und Liquiduslinie umschriebenen Bereich des Phasendiagramms der Ausgangsmischung gehalten wird. Weiterhin ist es im Fall eines Eutektikums bevorzugt, dass die Temperatur nicht unterhalb der Temperatur des S-L-Schnittpunkts des für die Extraktion ausgewählten Konzentrationsbereichs des Ausgangsmischungsbestandteils mit der höchsten Konzentration fällt.

Zudem ist es erfindungsgemäß bevorzugt, dass wenigstens ein Teil des Phasenbildners nach Bildung des Phasensystems, vorzugsweise nach Bildung des Kristallsystems, wiedergewonnen und in Schritt (b) der Ausgangsmischung wieder zugesetzt wird. Es kann beispielsweise in dem Fall, dass das Kristallsystem Kristalle des sauren Monomers aufweist, die mit dem Phasenbildner angereicherte wässrige Phase aus dem Phasensystem bzw. dem Kristallsystem entfernt werden und nach entsprechender Aufarbeitung der wasserabgereicherte Phasenbildner in Schritt (b) der Ausgangsmischung wieder zugesetzt werden.

Weiterhin ist es in dem erfindungsgemäßen Verfahren bevorzugt, dass das Phasensystem mindestens eine erste flüssige Phase und eine durch eine Phasengrenze von der ersten flüssigen Phase abgegrenzte mindestens eine weitere flüssige Phase als ein Phasensystem ausbildet. Bei der ersten flüssigen Phase handelt es sich um eine im Vergleich zur weiteren flüssigen Phase wasserarmen flüssigen Phase. Weiterhin ist es bevorzugt, dass die wasserarme flüssige Phase eine geringere Dichte besitzt als die wasserreiche flüssige Phase. Der Dichteunterschied beträgt vorzugsweise mindestens 10 kg/m³, vorzugsweise mindestens 100 kg/m³ und besonders bevorzugt mindestens 200 kg/m³. Zudem ist es in dem erfindungsgemäßen Verfahren bevorzugt, dass die Konzentration an saurem Monomer in der wasserarmen Phase im Vergleich zu der Konzentration an saurem Monomer in der wasserreichen Phase größer ist. Über die Trennung der verschiedenen flüssigen Phasen können die einzelnen Ausgangsmischungsbestandteile von einander abgetrennt werden.

Weiterhin ist es in dem erfindungsgemäßen Verfahren bevorzugt, dass die in einer Phase des Phasensystems sich bildenden Produktkristalle überwiegend, vorzugsweise zumindest 80 Gew.-% und besonders bevorzugt zumindest 90 Gew.-%, jeweils bezogen auf den Produktkristall, aus saurem Monomer oder aus Wasser bestehen.

Zur weiteren Steigerung der Aufreinigungsleistung des erfindungsgemäßen Verfahrens ist es bevorzugt, dass das Kristallsystem oder der abgetrennte Produktkristall oder beide einem weiteren Reinigungsschritt unterzogen werden. Als in diesem weiteren Reinigungsschritt oder Reinigungsschritten einsetzbare Verfahren und Vorrichtungen sind alle dem Fachmann geläufigen und geeignete Verfahren zu nennen. Unter diesen Verfahren sind diejenigen bevorzugt, die das zu reinigende Material thermisch wenig belasten. Hierzu gehören insbesondere Kristallisationsverfahren wie die Schichtkristallisation und die Suspensionskristallisation, wobei die Suspensionskristallisation bevorzugt ist. Kristallisationsprodukte der vorgenannten Kristallisationsverfahren können weiterhin einem Waschschritt unterzogen werden. Weiterhin kann es vorteilhaft sein, dass die Kristalle der Kristallphase vor der Überführung in den weiteren Reinigungsschritt mindestens teilweise aufgeschmolzen werden.

Geeignete Verfahren hierzu sind aus EP 0 616 998, WO 99/14181, US 4,780,568 sowie WO 02/055469 bekannt. Besonders geeignete Reinigungsverfahren und Reinigungsvorrichtungen für Produktkristalle, die mindestens 80 Gew.-%, bevorzugt mindestens 95 Gew.-% und besonders bevorzugt mindestens 99 Gew.-%, jeweils bezogen auf den Produktkristall, saures Monomer beinhalten, sind in WO 02/055469 offenbart.

Des weiteren ist es in dem erfindungsgemäßen Verfahren bevorzugt, dass das saure Monomer einen pH-Wert von weniger als 7, vorzugsweise weniger als 5 und besonders bevorzugt weniger als 3 besitzt. Als saure Monomere besonders bevorzugt ist (Meth)Acrylsäure. Die pH-Wert-Bestimmung erfolgt bei dem sauren Monomer und bei der Brönsted-Säure in wässriger Lösung nach "*Maßanalyse Theorie und Praxis der klassischen und der Elektrochemischen Titrierverfahren*" Walter de Gruyter & Co. Berlin 1969. Der Begriff "(Meth)Acrylsäure" wird in diesem Text für die Verbindung mit dem Nomenklaturnamen "Methacrylsäure" und "Acrylsäure" verwendet. Von beiden Verbindungen ist die Acrylsäure erfindungsgemäß bevorzugt.

Weiterhin betrifft die Erfindung eine Vorrichtung zur Synthese eines eine Doppelbindung aufweisenden, sauren Monomers, aufweisend in fluidleitender Verbindung als Komponenten:
i. als eine Monomersyntheseeinheit aufweisend eine Gasphasenmonomersyntheseeinheit oder eine Flüssigphasenmonomersyntheseeinheit,
ii. eine auf die Gasphasenmonomersyntheseeinheit folgende Quencheinheit,
iii. gegebenenfalls eine auf die Flüssigphasenmonomersyntheseeinheit oder auf die Quencheinheit folgende erste Reinigungseinheit,
iv. eine erste Extraktionseinheit, aufweisend als Bestandteile:
   (aa) eine mit der Flüssigphasenmonomersyntheseeinheit oder der Quencheinheit oder mit der gegebenenfalls vorliegenden ersten Reinigungseinheit verbundene Ausgangsmischungsführung,
   (bb) eine Phasenbildnerführung,
   (cc) einen die Ausgangsmischungsführung und die Phasenbildnerführung aufnehmenden Extraktionsbehälter,
v. gegebenenfalls eine weitere mit der ersten Extraktionseinheit verbundene weitere Extraktionseinheit oder weitere Reinigungseinheit oder beides.
wobei
- dei erste Extraktionseinheit einen von dem Extraktionsbehälter getrennten Kristallisationsbereich aufweist, wobei mindestens ein Teil des Kristallisationsbereichs kühlbar ist,
   oder
- ein Bereich des Extraktionsbehälters kühlbar ist,

In einer bevorzugten Ausführungsform der erfindungsgemäßen Vorrichtung ist ein Bereich des Extraktionsbehälters und besonders bevorzugt der Extraktionsbehälter kühlbar. Diese Ausgestaltung eignet sich besonders für die vorstehend beschriebene einstufige Durchführung des erfindungsgemäßen Verfahrens.

In einer anderen Ausführungsform der erfindungsgemäßen Vorrichtung weist diese neben einen Extraktionsbehälter, einen von diesem Extraktionsbehälter getrennten Kristallisanonsbereich auf. In diesem getrennten Kristallisationsbereich ist mindestens ein Teil dieses Bereichs kühlbar. Typische Kristallisationsbereiche sind die an anderer Stelle beschriebenen Kristallerzeuger. Diese Ausgestaltung der erfindungsgemäßen Vorrichtung eignet sich besonders für die vorstehend beschriebene zwei und mehrstufige Durchführung des erfindungsgemäßen Verfahrens.

Unter "fluidleitend" wird erfindungsgemäß verstanden, dass Gase oder Flüssigkeiten, Suspensionen eingeschlossen, oder deren Mischungen durch entsprechende Leitungen geführt werden. Hierzu lassen sich insbesondere Rohrleitungen, Pumpen und der gleichen einsetzen.

Gemäß einer bevorzugten erfindungsgemäßen Ausführungsform der Vorrichtung zur Synthese des sauren Monomers weist diese zwingend eine auf die Quencheinheit folgende erste Reinigungseinheit auf.

Gemäß einer weiteren bevorzugten erfindungsgemäßen Ausführungsform der Vorrichtung zur Synthese des sauren Monomers weist diese zwingend eine auf die Flüssigphasenmonomersyntheseeinheit folgende erste Reinigungseinheit auf

Gemäß einer anderen bevorzugten erfindungsgemäßen Ausführungsform der Vorrichtung zur Synthese eines sauren Monomers weist diese zwingend eine weitere mit der ersten Extraktionseinheit verbundene weitere Extraktionseinheit oder weitere Reinigungseinheit oder beides auf.

Daher ist es bei der erfindungsgemäßen Vorrichtung zur Synthese eines sauren Monomers bevorzugt, dass die Monomersyntheseeinheit mindestens zwei Reaktoren aufweist. Als Reaktoren kommen alle dem Fachmann für die Synthese von sauren Monomeren geeigneten Reaktoren in Betracht. In diesem Zusammenhang sind beispielsweise Röhrenreaktoren, Wandreaktoren und Schichtreaktoren zu nennen. Im Zusammenhang mit dem Reaktor ist es bevorzugt, dass der Reaktor einen geträgerten Übergangsmetalloxidkatalysator aufweist, der die vorzugsweise durch Gasphasenoxidation eines eine Doppelbindung aufweisenden Kohlenwasserstoffs erfolgende Synthese des sauren Monomers erleichtert. Die geträgerten Übergangsmetallkatalysatoren können auf allen dem Fachmann bekannten Materialien geträgert werden. In diesem Zusammenhang sind verschiedene Metalle, insbesondere gegenüber den Reaktionsbedingungen bei der Synthese des sauren Monomers inerte und keramische Materialien wie Stähle, Mischoxide und einzelne Oxide des Aluminiums, des Siliziums und des Titans, beispielsweise Rutil und Anatas zu nennen. Als Übergangsmetalloxidkatalysatoren sind insbesondere die Mischoxide des Vanadiums, des Nickels und Molybdäns oder eine Mischung aus mindestens zwei davon bevorzugt. In diesem Zusammenhang wird insbesondere auf die Ausführungen in DE 196 06 877 unter Stufe (I) als Teil dieser Offenbarung verwiesen.

Im Zusammenhang mit der erfindungsgemäßen Vorrichtung zur Synthese eines sauren Monomers ist es besonders bevorzugt, dass mindestens einer der Reaktoren ein Gasphasenreaktor ist.

Das in der Monomersyntheseeinheit durch die mindestens ein, vorzugsweise zwei Reaktoren durch die Edukte gebildete, das saure Monomer beinhaltende, meist gasförmige Reaktorprodukt wird anschließend in die Quencheinheit überführt. Bei der Quencheinheit handelt es sich in der Regel um einen Quenchturm, in dem gasförmiges Reaktorprodukt mit Wasser in Kontakt gebracht wird um auf diese Art und Weise das saure Monomer in eine flüssige Phase zu überführen.

In einer erfindungsgemäß bevorzugten Ausführungsform der Vorrichtung zur Synthese eines sauren Monomers folgt auf die Quencheinheit eine Reinigungseinheit in Form einer Destillationskolonne. In dieser Destillationskolonne wird das in der Quencheinrichtung entstandene Quenchprodukt durch Destillation in seine verschiedene Bestandteile, darunter das saure Monomer durch thermische Einwirkung weitestgehend aufgetrennt. Es ist weiterhin bevorzugt, dass die Destillationskolonne in ihrem unteren Bereich einen Kolonnensumpf aufweist, der mit der Ausgangsmischungsführung verbunden ist, um auf diese Art und Weise die Sumpf-Ausgangsmischung in die Extraktionseinheit zu überführen. Auf diese Weise können die im Zuge der Destillation anfallenden, nicht in den oberen Bereich der Destillationskolonne transportierten meist eine nicht unerhebliche Menge sauren Monomers beinhaltenden Sumpfgemische einer weiteren Aufreinigung unterzogen werden, die zu einer Steigerung der Effizienz der Vorrichtung und insbesondere zu einer Steigerung der Ausbeute an saurem Monomer führt.

Gemäß einer weiteren Ausführungsform der erfindungsgemäßen Vorrichtung zur Synthese eines sauren Monomers ist es bevorzugt, dass die Destillationskolonne in oberen Bereich einen Kolonnenkopf aufweist, der ebenfalls mit einer Ausgangsmischungsführung verbunden ist. Dieses dient dazu, die in den Kopfprodukten des Destillationsprozesses nach wie vor neben dem gewünschten sauren Monomer Verunreinigungen enthaltenden Mischungen als Kopf-Ausgangsgemisch einer Extraktionseinheit zur weiteren Aufreinigung und damit zur weiteren Effizienzsteigerung der Vorrichtung zur Synthese eines sauren Monomers zuzuführen.

Im Zuge der destillativen Aufarbeitung des Quenchprodukts kann es zur Bildung von Oligomeren des sauren Monomers kommen, die durch eine geeignete Oligomerenspaltvorrichtung in saure Monomere zurückgeführt werden müssen. Daher ist in einer erfindungsgemäßen Ausführungsform der Vorrichtung zur Synthese eines sauren Monomers anschließend an die erste Reinigungseinheit eine Spaltvorrichtung für Oligomere des sauren Monomers vorgesehen. Als Spaltvorrichtung kommen alle dem Fachmann für die Spaltung von sauren Monomeren geeignete Verfahren in Betracht, in diesem Zusammenhang sind insbesondere katalytische Dimeren- oder Trimerenspaltvorrichtungen zu nennen. Weiterhin ist es bevorzugt, dass die Spaltvorrichtung in ihrem unteren Bereich einen Spaltvorrichtungssumpf aufweist, der mit der Ausgangsmischungsführung verbunden ist, um auf diese Weise die Oligomeren-Ausgangsgemische der auf diese Weise die Oligomeren-Ausgangsgemische der Extraktionseinheit zuzuführen.

In einer anderen Ausführungsform der erfindungsgemäßen Vorrichtung zur Synthese eines sauren Monomers ist es bevorzugt, dass diese mit einer auf die Quencheinheit folgenden ersten Reinigungseinheit ausgestattet ist, wobei diese erste Reinigungseinheit ein Kristallerzeuger ist. Das durch den Kristallerzeuger kristallisierte Quenchprodukt bildet oftmals bei der Kristallisation ein eutektisches Gemisch, in dem das saure Monomer nicht in den gewünschten Menge abgetrennt werden kann. Daher ist es bevorzugt, dass über die Ausgangsmischungsführung das Eutektikum-Ausgangsgemisch in die Extraktionseinheit überführt wird, um dort durch das erfindungsgemäße Verfahren aufgereinigt zu werden. Kristallerzeuger können alle dem Fachmann bekannten und erfindungsgemäß geeigneten eingesetzt werden. Darunter fallen Schicht- und Suspensionskristallerzeuger. Bei den Suspensionskristallerzeugem können vorteilhafterweise Kesselkristallisatoren, Kratzkristallisatoren, Kühlscheibenkristallisatoren, Kristallisierschnecken, Trommelkristallisatoren, und dergleichen eingesetzt werden, wobei die Suspensionskristallerzeuger bevorzugt mit einer nachgeschalteten Waschkolonne betrieben werden. In diesem Zusammenhang wird auf die Offenbarung der WO 99/14181 verwiesen.

Gemäß einer Ausgestaltung des erfidungsgemässen Verfahrens wird ein eine Doppelbindung aufweisendes, saures Monomer hergestellt, indem, eine im Reaktor entstandene Synthesemischung beinhaltend das saure Monomer mit Wasser in Kontakt gebracht und gegebenenfalls nach mindestens einem weiteren Aufarbeitungsschritt als eine Ausgangsmischung einem erfindungsgemäßen Verfahren zur Aufreinigung eines sauren Monomers zugeführt wird.

Außerdem ist es bei dieser Ausgestaltung des erfindungsgemässen Verfahrens bevorzugt, dass das Verfahren in mindestens einem Reaktor durch Oxidation eines mindestens eine Doppelbindung aufweisenden Kohlenwasserstoffs durchgeführt wird. Bei diesen Kohlenwasserstoffen handelt es sich um C2- bis C10-, bevorzugt C3-bis C5- und besonders bevorzugt C3-Kohlenwasserstoff. Besonders bevorzugt als Kohlenwasserstoff ist Propylen im Fall der Herstellung von Acrylsäure.

Als Flüssigphasenmonomersyntheseeinheit kommen alle dem Fachmann bekannten und geeignet erscheinenden Systeme in Betracht, in dem ausgehend von einem eine Doppelbindung aufweisenden Kohlenwasserstoff als Edukt in der flüssigen Phase eines Lösemittels das saure Monomer durch Übergangsmetallverbindungen mit Ligandensystemen als Katalysatoren das saure Monomer, vorzugsweise bereits in einem Lösemittel gelöst, erhalten wird.

Ferner ist es bei dieser Ausgestaltung des erfindungsgemässen Verfahrens bevorzugt, dass eine erfindungsgemäße Vorrichtung zur Synthese eines sauren Monomers eingesetzt wird.

Die Erfindung wird nun an Hand von Zeichnungen und Beispielen in nicht limitierender Weise näher erläutert.
Fig.1 zeigt einen schematischen Aufbau einer erfindungsgemäßen Monomersynthesevorrichtung mit an verschiedenen Stellen angeordneten möglichen Extraktionseinheiten in Form eines Flussdiagramms.
Fig. 2 zeigt die Kombination einer erfindungsgemäßen Extraktionseinheit mit einer Phasenbildnerauibereitung und einer weiteren Reinigungseinheit als Flussdiagramm.
Fig. 3 zeigt eine weitere zweistufige Kombination einer Extraktionseinheit mit von einander getrennten Extraktions- und Kristallisationsbereich und einer Flüssigphasenmonomersyntheseeinheit als Flussdiagramm.

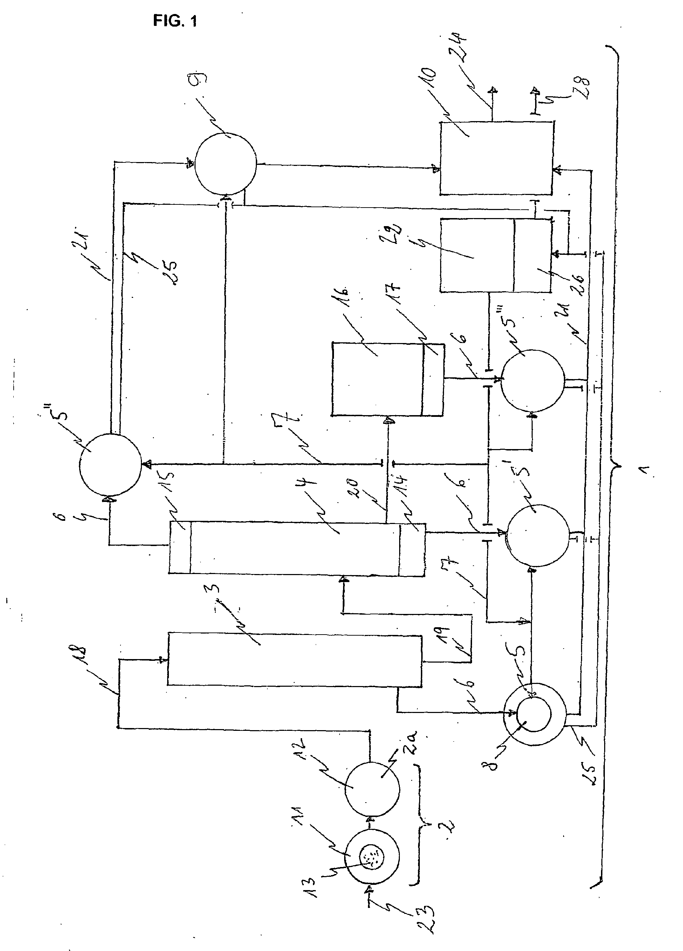

In der in Fig. 1 abgebildeten Monomersynthesevorrichtung 1 wird über einen Edukteinlass 23 das für die Synthese des sauren Monomers notwendige Edukt - im Fall der Synthese von Acrylsäure, Propylen und Sauerstoff - in einen ersten Reaktor 11 eingeleitet. Das Eduktgemisch wird über einen geträgerten Übergangsmetallkatalysator 13 durch eine Gasphasenoxidationsreaktion zunächst in dem ersten Reaktor 11 im Fall der Acrylsäuresynthese hauptsächlich zu Acrolein umgesetzt, um dann dieses in einem weiteren Reaktor 12 einer weiteren Gasphasenoxidation zur Acrylsäure zu unterziehen. Der erste Reaktor 11 und der weitere Reaktor 12 bilden eine Gasphasenmonomersyntheseeinheit 2a als Monomersyntheseeinheit 2. Über eine Reaktorproduktführung 18 wird das Reaktorprodukt aus der Monomersyntheseeinheit 2 in eine Quencheinheit 3 geführt. An die Quencheinheit 3 kann über eme.Ausgangsmischungsfuhrung 6 eine erste Extraktionseinheit 5 angeschlossen sein, in der das Quenchprodukt dem erfindungsgemäßen Aufarbeitungsverfahren unterzogen wird. Es ist weiterhin möglich, das Quenchprodukt über eine Quenchproduktführung 19 einer ersten Reinigungseinheit 4 zuzuführen. Bei der ersten Reinigungseinheit 4 kann es sich zum einen um eine Kristallisationsvorrichtung, eine Kristallisationsvorrichtung mit einer Waschvorrichtung oder um eine Destillationskolonne handeln. Für den Fall, dass es sich bei der ersten Reinigungseinheit 4 um eine Destillationskolonne handelt, weist diese Destillationskolonne im unteren Bereich einen Kolonnensumpf 14 und im oberen Bereich der Destillationskolonne einen Kolonnenkopf 15 auf. Sowohl an dem Kolonnensumpf 14 als auch an dem Kolonnenkopf 15 kann eine weitere erste Extraktionseinheit 5' bzw. 5" über entsprechende Ausgangsmischungsführungen 6 angeschlossen sein. Weiterhin ist es möglich, dass an die als Destillationskolonne ausgelegte erste Reinigungseinheit 4 über eine Oligomerenführung 20 an eine Spaltvorrichtung 16 für Oligomere des sauren Monomers angeschlossen ist. Diese Spaltvorrichtung 16 weist einen Spaltvorrichtungssumpf 17 auf, der durch eine weitere Ausgangsmischungsführung 6 mit einer weiteren Extraktionseinheit 5"' verbunden ist. Für den Fall, dass die erste Reinigungseinheit 4 eine Kristallisationsvorrichtung oder eine Kristallisationsvorrichtung mit einer Waschvorrichtung ist, ist es ferner möglich, dass die in der Kristallisationsvorrichtung oder in der Kristallisationsvorrichtung mit Waschvorrichtung anfallenden, das saure Monomer in aufgereinigter Form beinhaltenden Produkte, insbesondere wenn diese als wässrige Zusammensetzungen nahe dem eutektischen Punkt dieser Zusammensetzungen vorliegen, über die Ausgangsmischungsführung 6 einer weiteren ersten Extraktionseinheit 5' zugeführt werden. Die erste Extraktionseinheit 5 weist einen Extraktionsbehälter 8 auf, in dem neben der Ausgangsmischungsführung 6 eine Phasenbildnerführung 7 einmündet. Die so ausgestaltet Extraktionseinheit weist somit Phasentrennungs- und Kristallisationsbereich gemeinsam in dem Extraktionsbehälter 8 auf. Auch die weiteren ersten Extraktionseinheiten 5, 5', 5" sowie 5"' werden über die jeweiligen Phasenbildnerführungen 7 mit Phasenbildner aus einem Phasenbildnertank 22 versorgt. Über eine Produkt/Kristallführung 21 wird das in den ersten Extraktionseinheiten 5, 5', 5" sowie 5"' aufgereinigten Produkt einer weiteren Reinigungseinheit 10 zugeführt. Hierbei ist es möglich, dass die Produkt/Kristallführung 21 durch eine weitere Extraktionseinheit 9 unterbrochen wird, in der die Kristallphase einer weiteren erfindungsgemäßen Aufreinigung unterzogen wird. Als weitere Reinigungseinheit 10 können alle dem Fachmann bekannten und für die weitere Aufreinigung geeigneten Vorrichtungen eingesetzt werden. Als weitere Reinigungseinheit 10 sind insbesondere Kristallisationsvorrichtungen oder Kristallisationsvorrichtungen mit Waschvorrichtungen bevorzugt. Von der weiteren Reinigungseinheit 10 wird über einen Reinproduktauslass 24 das aufgereinigte saure Monomer aus der Monomersynthesevorrichtung 1 ausgetragen. Die sich in den ersten Extraktionseinheiten 5, 5', 5" sowie 5"' bildenden Rückstände können über eine Rückstandsführung 25 einer Phasenbildneraufbereitung 26 zugeführt werden, in der der Phasenbildner zurückgewonnen und in den Phasenbildnertank 23 überführt wird. Über eine Rückstandsabführung 28 werden bei der Aufarbeitung und Rückgewinnung des Phasenbildners anfallende Rückstände aus der Monomersynthesevorrichtung 1 äusgeschleust.

Folgende in der Monomersynthesevorrichtung 1 in Fig. 1 dargestellten Kombinationen sind im einzelnen als Ausführungsformen beispielsweise zur Verwendung in weiteren Monomersynthesevorrichtungen 1 bevorzugt: Die Kombination der Quencheinheit 3 mit der ersten Extraktionseinheit 5, die Kombination der ersten Reinigungseinheit 4 mit der ersten Extraktionseinheit 5' für den Fall, dass die erste Reinigungseinheit 4 eine Kristallisationsvorrichtung oder eine Kristallisationsvorrichtung mit einer Waschvorrichtung ist; die Kombination der ersten Reinigungseinheit 4 mit der ersten Extraktionseinheit 5' für den Fall, dass die erste Extraktionseinheit 4 eine Destillationskolonne mit einem Kolonnensumpf 14 ist; die Kombination der ersten Reinigungseinheit 4 mit der ersten Extraktionseinheit 5" für den Fall, dass die erste Reinigungseinheit 4 eine Destillationskolonne mit einem Kolonnenkopf 15 ist; die Kombination der ersten Reinigungseinheit 4 mit der ersten Extraktionseinheit 5' wie der weiteren ersten Extraktionseinheit 5" für den Fall, dass die Reinigungseinheit 4 eine Destillationskolonne mit einem Kolonnensumpf 14 und einem Kolonnenkopf 15 ist; die Kombination der ersten Reinigungseinheit 4 mit der Spaltvorrichtung 16, die einen Spaltvorrichtungssumpf 17 aufweist, für den Fall, dass die erste Reinigungseinheit 4 eine Destillationskolonne ist, in Verbindung der weiteren ersten Extraktionseinheit 5".

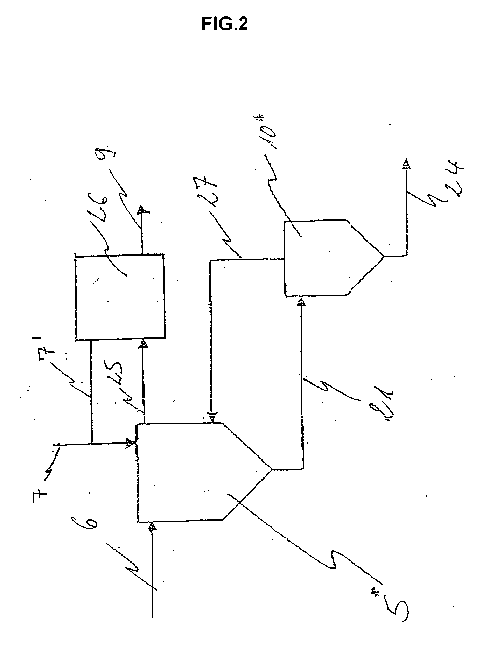

Fig. 2 zeigt eine vorzugsweise in einer Monomersynthesevorrichtung 1 an verschiedenen in Fig. 1 gezeigten Stellen einsetzbare Extraktionseinheit 5, wobei diese Extraktionseinheit 5 eine weitere Extraktionseinheit 5* aufweist, die mit einer weiteren Reinigungseinheit 10* kombiniert ist. Über die Ausgangsmischungszuführung 6 wird in die weitere Extraktionseinheit 5* die Ausgangsmischung zusammen mit dem über die Phasenbildnerführung 7 zugeführten Phasenbildner eingespeist, damit das in der Ausgangsmischung beinhaltete saure Monomer durch das erfindungsgemäße Verfahren aufgereinigt werden kann. Der Phasenbildner und möglichst wenig saures Monomer (weniger als 20 Gew.-%, bevorzugt weniger als 10 Gew.-% und besonders bevorzugt weniger als 5 Gew.-%, jeweils bezogen auf die durch die Rückstandsführung 25 ausgetragene Mischung) wird durch die Rückstandsführung 25 in die Phasenbildneraufbereitung 26 überführt. Der in der Phasenbildneraufbereitung 26 gewonnene Phasenbildner wird über die Phasenbildnerführung 7 der ersten Extraktionseinheit 5* wieder zugeführt. Über die Produkt/Kristallführung 21 wird die an saurem Monomer reiche Kristallphase in die weitere Reinigungseinheit 10* überführt. Bei dieser Reinigungseinheit 10* kann es sich zum einen um eine Kristallisationsvorrichtung oder zum anderen um eine Kristallisationsvorrichtung mit einer Waschvorrichtung handeln. Es kann bevorzugt sein, dass vor der Einleitung der Produkt/Kristallphase in die weitere Reinigungseinheit 10 die Kristalle des sauren Monomers mindestens teilweise aufgeschmolzen werden. In der weiteren Reinigungseinheit 10* wird das saure Monomer der Kristallphase weiter aufgereinigt, wobei die Rückstände dieser Reinigung über die Reinigungsrückstandsführung 27 wieder in die erste Extraktionseinheit 5* zurückgeführt werden können und das aufgereinigte saure Monomer über den Reinproduktauslass 24 aus der weiteren Reinigungseinheit 10* ausgeschleust werden kann.

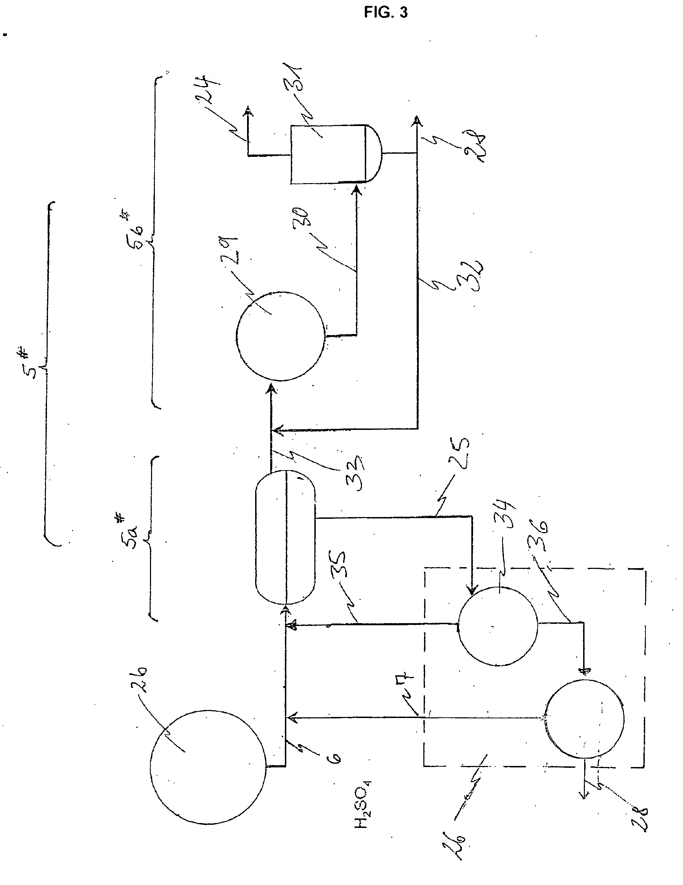

In Fig. 3 wird die beispielsweise in einer Flüssigphasenmonomersyntheseeinheit 2b erhaltenen Ausgangsmischung über die Ausgangsmischungsführung 6 mit im Fall der Aufreinigung von Acrylsäure im Bereich von 60 bis 80 Gew.-% Acrylsäure in eine Extraktionseinheit 5# geführt. Die Extraktionseinheit 5# weist einen Phasentrennungsbereich 5a# auf, von dem die produktreichere Phase, die vorzugsweise phasenbildnerärmer ist, über eine Produktphasenführung 33 in einen von dem Phasentrennbereich 5a# getrennten Kristallisationsbereich 5b# mit einem Kristaller 29 zur Erzeugung einer Kristallsuspension geleitet wird. Über eine Kristallsuspensionsführung 30 gelangt die Kristallsuspension in eine Waschkolonne 31, in der das Produkt abgetrennt und über den Reinproduktauslass 24 abgeführt und ggf. einer weiteren Reinigungseinheit 10 zugeführt werden kann. Über eine Mutterlaugenführung 32 kann die bei der Trennung in der Waschkolonne 31 anfallende Mutterlaugen in den Kristaller 29 zurückgeführt oder zumindest teilweise über die Rückstandsabführung 28 entsorgt werden. Über die Rückstandsführung 25 kann der produktarme und phasenbildnerreiche Rückstand aus dem Phasentrennbereich 5a# der Phasenbildneraufbereitung 26 zugeführt und der dort aufbereitete Phasenbildner - bevorzugt Schwefelsäure - von dort aus in die Phasenbildnerführung 7 eingespeist und der bei der Aufbereitung des Phasenbildners angefallene Rückstand über die Rückstandsabführung 28 entsorgt werden. Vorzugsweise weist die Phasenbildneraufbereitung 26 einen mit der verbrauchten Phasenbildner und weiter Rückstände führenden Rückstandsführung 25 verbundenen ersten Flashbehälter 34 auf. In dem ersten Flashbehälter 34 werden die leichtsiedenden Bestandteile, meist Wasser und Acrylsäure, des Rückstands verdampft und über die Wertstoffführung 35 dem Phasentrennungsbereich 5a# wieder zugeleitet. Der meist Phasenbildner und Rückstand enthaltenden Schwersiederanteil wird über eine Schwersiederführung 36 einem, meist bei höheren Temperaturen betriebenen, weitem Flashbehälter 37 zugeführt. In diesem weiteren Flashbehälter 37 wird der Phasenbildner als Leichtsieder abgetrennt und über die Phasenbildnerführung 7 dem Phasenbildungsbereich 5a# zugeleitet. Der schwersiedende Anteil wird über eine Rückstandsabführung 28 ausgeschleust.

### BEISPIELE:

### 1. PHASENTRENNUNG A

In einem auf 20 °C temperierten doppelwandigen Laborglasgefäß mit einem Fassungsvermögen 1,5 1 wurden 724g eines Gemisches mit einer Zusammensetzung gemäß Tabelle 1 vorgelegt und mit einem Magnetrührer gerührt. Anschließend wurden 226g Phasenbildner (96%-ige Schwefelsäure) zugesetzt. In Folge der Zugabe wurde eine Phasentrennung beobachtet. Die Zusammensetzung der einzelnen Phasen sind in Tabellen 1 angegeben.

**Tabelle 1**

| | Edukt | obere Phase | untere Phase |
|---|---|---|---|
| | Gehalt in Masse % | Gehalt in Masse % | Gehalt in Masse % |
| Acrolein | 0,025 | <0,01 | <0,01 |
| Essigsäure | 3,146 | 3,091 | 1,994 |
| Furfural | 0,024 | 0,023 | <0,01 |
| Benzaldehyd | 0,028 | 0,042 | <0,01 |
| Propionsäure | 0,013 | 0,036 | 0,042 |
| Protoanemonin | 0,017 | 0,019 | <0,01 |
| Acrylsäure | 65,925 | 93,100 | 25,400 |
| Dimerer Acrylsäure | 0,388 | 0,777 | 0,353 |
| Maleinsäureanhydrid | 0,100 | 0,422 | 0,529 |
| Wasser | 30,200 | 3,700 | 36,6 |
| Schwefelsäure | - | 2,900 | 36,3 |

### 2. PHASENTRENNUNG B

Der Versuch zu Phasentrennung A wurde mit dem Unterschied wiederholt, indem 874 g eines Gemisches mit der Zusammensetzung gemäß Tabelle 2 und 305 g 70%-ige wässrige Natriumhydrogensulfatlösung als Phasenbildner eingesetzt wurden. Die Zusammensetzung der entstehenden Phasen ist in Tabelle 2 angegeben.

**Tabelle 2**

| | Edukt | obere Phase | untere Phase |
|---|---|---|---|
| | Gehalt in Masse % | Gehalt in Masse % | Gehalt in Masse % |
| Acrolein | 0,025 | 0,011 | <0,001 |
| Essigsäure | 3,146 | 3,400 | 0,700 |
| Furfural | 0,024 | 0,023 | 0,001 |
| Benzaldehyd | 0,028 | 0,026 | <0,001 |
| Propionsäure | 0,013 | 0,018 | 0,009 |
| Protoanemonin | 0,017 | n.a. | n.a. |
| Acrylsäure | 65,925 | 68,100 | 8,000 |
| Dimerer Acrylsäure | 0,388 | 0,500 | <0,001 |
| Maleinsäureanhydrid | 0,100 | 0,600 | 0,300 |
| Wasser | 30,200 | 23,700 | 47,600 |
| Natriumhydrogensulfat | - | 8,700 | 43,000 |

### 3. EXTRAKTIONS-KRISTALLISATIONSVERSUCHE (EINSTUFIG)

In einem auf 20 °C temperierten doppelwandigen Laborglasgefäß mit einem Fassungsvermögen 250 ml wurden 170 g eines Gemisches mit einer Zusammensetzung gemäß Tabelle 3 (Edukt) mit einem Magnetrührer gerührt. Anschließend wurden 55g einer 96 %-igen Schwefelsäure als Phasenbildner unter Ausbildung von zwei Phasen zugesetzt. Anschließend wurde die Temperatur so lange reduziert bis Kristalle bei 6,8°C sich bildeten. Die Temperatur wurde weiter auf 5,4°C bis zum Abschluß der Kristallbildung reduziert. Die Kristalle der so entstandenen Kristallphase wurde mittels eines Vakuumnutschfilters abgetrennt und mit 102g 99,8 %-iger Acrylsäure gewaschen. Die verschiedenen Zusammensetzungen sind in Tabelle 3 angegeben. Die Kristallisation aus der vornehmlich Acrylsäure enthaltenden Phase war so vollständig, dass sich ein Rückstand in Form einer wässrigen/organischen Suspension bildete. Diese Suspension konnte leicht von den einen Filterkuchen bildenden Kristallen mit dem Vakuumnutschfilter abgetrennt werden. Es wurden nach der Wäsche mit 99,8 %-iger Acrylsäure Kristalle mit einer Ausbeute von 51 %, bezogen auf die im Edukt eineingesetzte Acrylsäure, erhalten.

**Tabelle 3**

| Bezeichnung | Einheit | Edukt | Kristalle nach Wäsche | Kristalle vor Wäsche | Rückstand (wässrige/org. Phase) |
|---|---|---|---|---|---|
| Wasser | Gew.-% | 33,7 | 0,8 | 4,4 | 39,3 |
| Acrylsäure | Gew.-% | 62,6 | 97,6 | 83,3 | 24,5 |
| Essigsäure | Gew.-% | 3,0 | 0,2 | 1,2 | 2,5 |
| Propionsäure | Gew.-% | 0,012 | <0,01 | 0,014 | 0,06 |
| Dimerer Acrylsäure | Gew.-% | 0,277 | 11 mg/kg | 0,3 | 0,2 |
| Maleinsäureanhydrid | Gew.-% | 0,17 | 38 mg/kg | 0,1 | 0,5 |
| Furfural | Gew.-% | 0,0221 | <0,01 | <0,01 | 0,038 |
| Benzaldehyd | Gew.-% | 0,0272 | <0,01 | 0,021 | 0,017 |
| Acrolein | Gew.-% | 0,033 | <0,01 | 0,01 | 0,014 |
| Protoanemonin | Gew.-% | 0,0158 | <0,01 | <0,01 | 0,018 |
| Schwefelsäure | Gew.-% | - | 1,3 | 2,3 | 34,5 |

### 4. KRISTALLISATION/SCHICHTKRISTALLISATION (ZWEISTUFIG)

In einem auf 10 °C temperierten doppelwandigen Laborglasgefäß mit einem Fassungsvermögen 41 wurden 21 eines analog zu Beispiels 1 als obere Phase gewonnen Edukts vorgelegt. Die Zusammensetzung des Edukts ergibt sich aus Tabelle 4. In die Mitte des Laborglasgefäßes wurde ein Kühlfinger mit einer Temperatur von 10°C eingebracht, wobei der Kühlfinger zuvor kurz in 99,8 %-ige Acrylsäure zur Benetzung der Kühlfingeroberfläche getaucht wurde. Anschließend wurde der Kühlfinger und das doppelwandige Laborgefäß auf eine Temperatur von 4°C abgekühlt, so dass sich auf der des Kühlfingeroberfläche eine Kristallschicht auszubilden begann. Der Kühlfinger wurde mit 0,15°C/min auf -8°C weiter abgekühlt. Die an Acrylsäure verarmte Mutterlauge wurde abgelassen und die Kristalle des Kühlfingers analysiert. Anschließend wurde der Kühlfinger mit 0,2°C/min auf 2°C erwärmt, um die auf dem Kühlfinger befindliche Kristallschicht durch Schwitzen weiter aufzureinigen. Die Zusammensetzungen der Kristalle der einzelnen Schritte sind in Tabelle 4 angegeben.

**Tabelle 4**

| | Summe | | Schwefelsäure | Acrylsäure | Dimerer Acrylsäure | Essig säure | Maleinsäure | Furfural | Benzalde hyd | Propionsäure | Wasser |
|---|---|---|---|---|---|---|---|---|---|---|---|
| Fraktionen | Masse (g) | Bestimmung | w (%) | w (%) | w (%) | w (%) | w (%) | w (%) | w (%) | w (%) | w (%) |
| Feed | 1982 | Analyse | 2,000 | 88,000 | 1,400 | 2,400 | 0,400 | 0,020 | 0,044 | 0,020 | 11,700 |
| Mutterlauge | 1891 | Bilanziert | 2,034 | 88,357 | 1,427 | 2,436 | 0,408 | 0,02 | 0,045 | 0,02 | 11,908 |
| Kristallisat vor Schwitzen | 82 (bilan ziert) | Bilanziert | 1,433 | 89,426 | 0,922 | 1,822 | 0,250 | 0,012 | 0,019 | 0,018 | 8,174 |
| Schwitzfraktion | 67 | Analyse | 1,700 | 88,000 | 1,100 | 2,000 | 0,300 | 0,014 | .0,023 | 0,019 | 9,600 |
| Kristallisat nach Schwitzen | 14 | Analyse | 0,200 | 96,000 | 0,100 | 1,000 | 0,020 | 0,001 | 0,002 | 0,014 | 1,600 |

Die Vorstehenden Angaben wurden mittels Gaschromatographie und HPLC ermittelt.

## Patentansprüche

1. Verfahren zur Aufreinigung eines eine Doppelbindung aufweisenden, sauren Monomers, aufweisend die Schritte
(a) Bereitstellen einer Ausgangsmischung, beinhaltend als Ausgangsmischungsbestandteile, jeweils bezogen auf die Ausgangsmischung,
(a1) mindestens 5 Gew.-% des sauren Monomers, und entweder
(a2) mindestens 0,01 Gew.-% Wasser, oder
(a3) mindestens 0,01 Gew.-% mindestens einer Ausgangsmischungskomponente, oder
(a2) und (a3),
wobei die Summe der Gew.-%-Anteile der Ausgangsmischungsbestandteile jeweils 100 Gew.-% ergibt;
(b) Zugabe eines Phasenbildners oder ein Salz dieses Phasenbildners oder eine Mischung von beiden unter Erhalt eines Reinigungsgemisches, woraus sich
(c) mindestens eine erste Phase und eine durch eine Phasengrenze von der ersten Phase abgegrenzte mindestens eine weitere Phase als ein Phasensystem ausbildet;
(d) Absenken der Temperatur des Phasensystems; wobei
(e) in einer der Phasen des Phasensystems ein mindestens 50 Gew.-% eines der Ausgangsmischungsbestandteile beinhaltender Produktkristall neben einem andern Ausgangsmischungsbestandteil als ein Kristallsystem entsteht;
(f) Abtrennen der Produktkristalle.

2. Verfahren nach Anspruch 1, wobei die Temperatur nur in einer Phase des Phasensystems abgesenkt wird.

3. Verfahren nach Anspruch 2, wobei die Temperatur in der monomerreichsten Phase des Phasensystems abgesenkt wird.

4. Verfahren nach einem der vorhergehenden Ansprüche, wobei das saure Monomer einen pH-Wert von weniger als 7 hat.

5. Verfahren nach einem der vorhergehenden Ansprüche, wobei das saure Monomer (Meth)Acrylsäure ist.

6. Verfahren nach einem der vorhergehenden Ansprüche, wobei der Phasenbildner eine Brönsted-Säure mit einen pH-Wert von weniger als 6 oder ein Salz einer Brönsted-Säure oder eine Mischung daraus ist.

7. Verfahren nach Anspruch 6, wobei die Brönsted-Säure Schwefelsäure oder eines ihrer Salze oder eine Mischung daraus ist.

8. Verfahren nach einem der vorhergehenden Ansprüche, wobei der Phasenbildner bei der Zugabe flüssig ist.

9. Verfahren nach einem der vorhergehenden Ansprüche, wobei das Reinigungsgemisch den Phasenbildner in einer Menge im Bereich von 1 bis 80 Gew.-%, bezogen auf das Reinigungsgemisch, beinhaltet.

10. Verfahren nach einem der vorhergehenden Ansprüche, wobei mindestens ein Teil des Phasenbildners nach Bildung des Phasensystems wiedergewonnen und in Schritt (b) der Ausgangsmischung wieder zugesetzt wird.

11. Verfahren nach einem der vorhergehenden Ansprüche, wobei das Kristallsystem oder der abgetrennte Monomerkristall oder beide mindestens einem weiteren Reinigungsschritt unterzogen werden.

12. Verfahren nach einem der Ansprüche 1 bis 11, wobei ein eine Doppelbindung aufweisendes, saures Monomer hergestellt wird, indem eine das saure Monomer und mindestens einen Synthesebestandteil beinhaltende aus mindestens einem Reaktor stammende Synthesemischung mit Wasser in Kontakt gebracht und, gegebenenfalls nach mindestens einem weiteren Aufarbeitungsschritt, als eine Ausgangsmischung einem Verfahren nach einem der Ansprüche 1 bis 11 zugeführt wird.

13. Verfahren nach Anspruch 12, wobei das saure Monomer in dem mindestens einen Reaktor durch Oxidation eines mindestens eine Doppelbindung aufweisenden Kohlenwasserstoffs erhalten wird.

14. Verfahren nach einem der Ansprüche 12 oder 13, wobei eine Vorrichtung (1) nach einem der Ansprüche 15 bis 24 zur Durchführung des Verfahrens verwendet wird.

15. Vorrichtung zur Synthese eines eine Doppelbindung aufweisenden, sauren Monomers (1), aufweisend in fluidleitender Verbindung als Komponenten:
i. als eine Monomersyntheseeinheit (2) aufweisend eine Gasphasenmonomersyntheseeinheit (2a) oder eine Flüssigphasenmonomersyntheseeinheit (2b),
ii. eine auf die Gasphasenmonomersyntheseeinheit (2a) folgende Quencheinheit (3),
iii. gegebenenfalls eine auf die Flüssigphasenmonomersyntheseeinheit (2b) oder auf die Quencheinheit (3) folgende erste Reinigungseinheit (4),
iv. eine erste Extraktionseinheit (5), aufweisend als Bestandteile:
(aa) eine mit der Flüssigphasenmonomersyntheseeinheit (2b) oder der Quencheinheit (3) oder mit der gegebenenfalls vorliegenden ersten Reinigungseinheit (4) verbundene Ausgangsmischungsführung (6),
(bb) eine Phasenbildnerführung (7),
(cc) einen die Ausgangsmischungsführung (6) und die Phasenbildnerführung (7) aufnehmenden Extraktionsbehälter (8),
wobei
- die erste Extraktionseinheit einen von dem Extraktionsbehälter getrennten Kristallisationsbereich aufweist, wobei mindestens ein Teil des Kristallisationsbereiches kühlbar ist,
oder
- ein Bereich des Extraktionsbehälters kühlbar ist,
v. gegebenenfalls eine weitere mit der ersten Extraktionseinheit (5) verbundene weitere Extraktionseinheit (9) oder weitere Reinigungseinheit (10) oder beides.

16. Vorrichtung nach Anspruch 15, wobei die Gasphasenmonomersyntheseeinheit (2a) mindestens einen Reaktor (11, 12) aufweist.

17. Vorrichtung nach Anspruch 16, wobei mindestens einer der Reaktoren einen geträgerten Übergangsmetalloxidkatalysator (13) aufweist.

18. Vorrichtung nach einem der Ansprüche 16 oder 17, wobei mindestens einer der Reaktoren ein Gasphasenreaktor ist.

19. Vorrichtung nach einem der Ansprüche 15 bis 18 mit der auf die Quencheinheit (3) folgenden ersten Reinigungseinheit (4), wobei die erste Reinigungseinheit (4) eine Destillationskolonne aufweist.

20. Vorrichtung nach Anspruch 19, wobei die Destillationskolonne in ihrem unteren Bereich einen Kolonnensumpf (14) aufweist, der mit der Ausgangsmischungsführung (6) verbunden ist.

21. Vorrichtung nach Anspruch 20, wobei die Destillationskolonne in ihrem oberen Bereich einen Kolonnenkopf (15) aufweist, der mit der Ausgangsmischungsführung (6) verbunden ist.

22. Vorrichtung nach einem der Ansprüche 15 bis 21, wobei sich an die erste Reinigungseinheit (4) eine Spaltvorrichtung (16) für Oligomere des sauren Monomers anschließt.

23. Vorrichtung nach Anspruch 22, wobei die Spaltvorrichtung (16) in ihrem unteren Bereich einen Spaltvorrichtungssumpf (17) aufweist, der mit der Ausgangsmischungsführung (6) verbunden ist.

24. Vorrichtung nach einem der Ansprüche 15 bis 23 mit der auf die Quencheinheit (3) folgenden ersten Reinigungseinheit (4), wobei die erste Reinigungseinheit (4) ein Kristallerzeuger ist.

## Claims

1. Process for purification of an acidic monomer having a double bond, comprising the steps of
(a) providing a starting mixture comprising as starting mixture constituents, respectively based on the starting mixture,
(a1) at least 5% by weight of the acidic monomer, and either
(a2) at least 0.01% by weight of water, or (a3) at least 0.01% by weight of at least one starting mixture component, or
(a2) and (a3),
where the sum of the percent by weight fractions of the starting mixture constituents is in each case 100% by weight;
(b) adding a phase former or a salt of this phase former or a mixture of both to obtain a purification mixture, from which
(c) at least one first phase and an at least one further phase, delimited from the first phase by a phase boundary, form as a phase system;
(d) lowering the temperature of the phase system; where
(e) in one of the phases of the phase system a product crystal comprising at least 50% by weight of one of the starting mixture constituents is formed in addition to another starting mixture constituent as a crystal system;
(f) isolating the product crystals.

2. Process according to Claim 1, wherein the temperature is lowered only in one phase of the phase system.

3. Process according to Claim 2, wherein the temperature is lowered in the most monomer-rich phase of the phase system.

4. Process according to any of the preceding claims, wherein the acidic monomer has a pH value of less than 7.

5. Process according to any of the preceding claims, wherein the acidic monomer is (meth)acrylic acid.

6. Process according to any of the preceding claims, wherein the phase former is a Brønsted acid with a pH value of less than 6 or a salt of a Brønsted acid or a mixture thereof.

7. Process according to Claim 6, wherein the Brønsted acid is sulphuric acid or one of its salts or a mixture thereof.

8. Process according to any of the preceding claims, wherein the phase former is liquid at the time of addition.

9. Process according to any of the preceding claims, wherein the purification mixture contains the phase former in a quantity in the range from 1% to 80% by weight, based on the purification mixture.

10. Process according to any of the preceding claims, wherein at least one part of the phase former is recovered after formation of the phase system and is added to the starting mixture again in step (b).

11. Process according to any of the preceding claims, wherein the crystal system or the isolated monomer crystal or both are subjected to at least one further purification step.

12. Process according to any of Claims 1 to 11, wherein an acidic monomer having a double bond is prepared by contacting a synthesis mixture, comprising the acidic monomer and at least one synthesis constituent and originating from at least one reactor, with water and supplying this mixture, where appropriate after at least one further workup step, as a starting mixture to a process according to any of Claims 1 to 11.

13. Process according to Claim 12, wherein the acidic monomer is obtained in the at least one reactor by oxidation of a hydrocarbon containing at least one double bond.

14. Process according to either of Claims 12 and 13, wherein a device (1) according to any of Claims 15 to 24 is used to carry out the process.

15. Device for the synthesis of an acidic monomer (1) having a double bond, having in fluid-conducting association as components:
i. a monomer synthesis unit (2) having a gas phase monomer synthesis unit (2a) or a liquid phase monomer synthesis unit (2b),
ii. a quench unit (3) following the gas phase monomer synthesis unit (2a),
iii. optionally a first purification unit (4) following the liquid phase monomer synthesis unit (2b) or the quench unit (3),
iv. a first extraction unit (5) having as constituents:
(aa) a starting mixture conduit (6) connected to the liquid phase monomer synthesis unit (2b) or to the quench unit (3) or to the optionally present first purification unit (4),
(bb) a phase former conduit (7),
(cc) an extraction container (8) accommodating the starting mixture conduit (6) and the phase former conduit (7),
where
- the first extraction unit has a crystallization portion which is separate from the extraction container, and at least part of the crystallization portion can be cooled,
or
- a portion of the extraction container can be cooled,
v. optionally, also connected to the first extraction unit (5), a further extraction unit (9) or further purification unit (10), or both.

16. Device according to Claim 15, wherein the gas phase monomer synthesis unit (2a) has at least one reactor (11, 12).

17. Device according to Claim 16, wherein at least one of the reactors has a supported transition metal oxide catalyst (13).

18. Device according to either of Claims 16 and 17, wherein at least one of the reactors is a gas phase reactor.

19. Device according to any of Claims 15 to 18 with the first purification unit (4) following the quench unit (3), the first purification unit (4) having a distillation column.

20. Device according to Claim 19, wherein the distillation column in its bottom portion has a column bottom (14) which is connected to the starting mixture conduit (6).

21. Device according to Claim 20, wherein the distillation column in its upper portion has a column head (15) which is connected to the starting mixture conduit (6).

22. Device according to any of Claims 15 to 21, wherein the first purification unit (4) is followed by a cracking device (16) for oligomers of the acidic monomer.

23. Device according to Claim 22, wherein the cracking device (16) has in its lower portion a cracking device bottom (17) which is connected to the starting mixture conduit (6).

24. Device according to any of Claims 15 to 23 with the first purification unit (4) following the quench unit (3), the first purification unit (4) being a crystal generator.

## Revendications

1. Procédé de purification d'un monomère acide comportant une double liaison, comprenant les étapes consistant à :
(a) préparer un mélange initial, contenant en tant que constituants du mélange initial, à chaque fois par rapport au mélange initial,
(a1) au moins 5 % en poids du monomère acide et soit
(a2) au moins 0,01 % en poids d'eau, soit
(a3) au moins 0,01 % en poids d'au moins un composant du mélange initial, soit
(a2) et (a3),
la somme des % en poids des constituants du mélange initial étant à chaque fois de 100 % en poids ;
(b) ajouter un séparateur de phases ou un sel de ce séparateur de phases ou un mélange des deux pour obtenir un mélange de purification,
(c) au moins une première phase et au moins une phase supplémentaire séparée de la première phase par une interface se formant en tant que système de phases ;
(d) diminuer la température du système de phases ;
(e) un produit cristallin contenant au moins 50 % d'un des constituants du mélange initial se formant dans une phase du système de phases à côté d'un autre constituant du mélange initial en tant que système cristallin ;
(f) séparer les produits cristallins.

2. Procédé selon la revendication 1, dans lequel la température n'est diminuée que dans une phase du système de phases.

3. Procédé selon la revendication 2, dans lequel la température est diminuée dans la phase la plus riche en monomère du système de phases.

4. Procédé selon l'une quelconque des revendications précédentes, dans lequel le monomère acide a une valeur de pH inférieure à 7.

5. Procédé selon l'une quelconque des revendications précédentes, dans lequel le monomère acide est l'acide (méth)acrylique.

6. Procédé selon l'une quelconque des revendications précédentes, dans lequel le séparateur de phases est un acide de Brönsted ayant une valeur de pH inférieure à 6 ou un sel d'un acide de Brönsted ou un de leurs mélanges.

7. Procédé selon la revendication 6, dans lequel l'acide de Brönsted est l'acide sulfurique ou un de ses sels ou un de leurs mélanges.

8. Procédé selon l'une quelconque des revendications précédentes, dans lequel le séparateur de phases est liquide lors de l'ajout.

9. Procédé selon l'une quelconque des revendications précédentes, dans lequel le mélange de purification contient le séparateur de phases en une quantité dans la plage allant de 1 à 80 % en poids, par rapport au mélange de purification.

10. Procédé selon l'une quelconque des revendications précédentes, dans lequel au moins une partie du séparateur de phases est récupérée après la formation du système de phases et de nouveau ajoutée au mélange initial lors à l'étape (b).

11. Procédé selon l'une quelconque des revendications précédentes, dans lequel le système cristallin ou le monomère cristallin séparé ou les deux sont soumis à au moins une étape de purification supplémentaire.

12. Procédé selon l'une quelconque des revendications 1 à 11, dans lequel un monomère acide comportant une double liaison est fabriqué en mettant en contact un mélange de synthèse, issu d'au moins un réacteur, contenant le monomère acide et au moins un constituant de synthèse, avec de l'eau et, éventuellement après au moins une étape de traitement supplémentaire, introduit en tant que mélange initial dans un procédé selon l'une quelconque des revendications 1 à 11.

13. Procédé selon la revendication 12, dans lequel le monomère acide est obtenu dans le ou les réacteurs par oxydation d'un hydrocarbure comportant au moins une double liaison.

14. Procédé selon l'une quelconque des revendications 12 ou 13, dans lequel un dispositif (1) selon l'une quelconque des revendications 15 à 24 est utilisé pour la réalisation du procédé.

15. Dispositif de synthèse d'un monomère acide comportant une double liaison (1), comportant en tant que composants en connexion fluidique :
i. une unité de synthèse de monomère (2) comportant une unité de synthèse de monomère en phase gazeuse (2a) ou une unité de synthèse de monomère en phase liquide (2b),
ii. une unité de refroidissement (3) suivant l'unité de synthèse de monomère en phase gazeuse (2a),
iii. éventuellement une première unité de purification (4) suivant l'unité de synthèse de monomère en phase liquide (2b) ou l'unité de refroidissement (3),
iv. une première unité d'extraction (5), comportant en tant que constituants :
(aa) une conduite pour mélange initial (6) reliée avec l'unité de synthèse de monomère en phase liquide (2b) ou l'unité de refroidissement (3) ou avec la première unité de purification éventuellement présente (4),
(bb) une conduite pour séparateur de phases (7), (cc) un contenant d'extraction (8) qui loge la conduite pour mélange initial (6) et la conduite pour séparateur de phases (7),
- la première unité d'extraction comportant une zone de cristallisation séparée du contenant d'extraction, au moins une partie de la zone de cristallisation étant refroidissable
ou
- une zone du contenant d'extraction étant refroidissable,
v. éventuellement une unité d'extraction supplémentaire (9) ou une unité de purification supplémentaire (10) ou les deux, reliées avec la première unité d'extraction (5).

16. Dispositif selon la revendication 15, dans lequel l'unité de synthèse de monomère en phase gazeuse (2a) comporte au moins un réacteur (11, 12).

17. Dispositif selon la revendication 16, dans lequel au moins un des réacteurs comporte un catalyseur supporté à base d'oxyde de métal de transition (13).

18. Procédé selon l'une quelconque des revendications 16 ou 17, dans lequel au moins un des réacteurs est un réacteur en phase gazeuse.

19. Dispositif selon l'une quelconque des revendications 15 à 18 avec la première unité de purification (4) suivant l'unité de refroidissement (3), dans lequel la première unité de purification (4) comporte une colonne de distillation.

20. Dispositif selon la revendication 19, dans lequel la colonne de distillation comporte un fond de colonne (14) dans sa partie inférieure, qui est relié avec la conduite pour mélange initial (6).

21. Dispositif selon la revendication 20, dans lequel la colonne de distillation comporte une tête de colonne (15) dans sa partie supérieure, qui est reliée avec la conduite pour mélange initial (6).

22. Dispositif selon l'une quelconque des revendications 15 à 21, dans lequel un dispositif de partage (16) pour les oligomères du monomère acide est connecté à la première unité de purification (4).

23. Dispositif selon la revendication 22, dans lequel le dispositif de partage (16) comporte un fond de dispositif de partage (17) dans sa partie inférieure, qui est relié avec la conduite pour mélange initial (6).

24. Dispositif selon l'une quelconque des revendications 15 à 23 avec la première unité de purification (4) suivant l'unité de refroidissement (3), dans lequel la première unité de purification (4) est un générateur de cristaux.
